(19) Europäisches Patentamt
European Patent Office
Office européen des brevets

(11) Publication number: 0 225 409 A1

(12) EUROPEAN PATENT APPLICATION

(21) Application number: 85308772.4

(22) Date of filing: 02.12.85

(51) Int. Cl.⁴: A61K 43/00, C07F 9/38, C07B 59/00

(43) Date of publication of application: 16.06.87 Bulletin 87/25

(84) Designated Contracting States: BE CH DE FR GB IT LI NL SE

(71) Applicant: THE DOW CHEMICAL COMPANY
2030 Dow Center Abbott Road P.O. Box 1967
Midland, MI 48640(US)

(72) Inventor: Simon, Jaime
Route 1, Box 120-G Angleton
Texas 77515(US)
Inventor: Volkert, Wynn A.
2203 Garden Drive Columbia
Missouri 65212(US)
Inventor: Wilson, David A.
229 San Saba Richwood
Texas 77531(US)
Inventor: Troutner, David E.
402 Edgewood Avenue Columbia
Missouri 65201(US)
Inventor: Goeckeler, William F.
617 Hemlock Midland
Michigan 48640(US)

(74) Representative: Raynor, John et al
W.H. Beck, Greener & Co 7 Stone Buildings
Lincoln's Inn
London WC2A 3SZ(GB)

(54) **Organic amine phosphonic acid complexes for the treatment of calcific tumors.**

(57) Particle-emitting radionuclides (Y-90 and In-115) have been complexed with phosphonic acid derivatives of organic amine or substituted organic amine compounds wherein the nitrogen and phosphorus are interconnected by an alkylene group. These complexes have been found useful in the treatment of calcific tumors in animals.

EP 0 225 409 A1

# ORGANIC AMINE PHOSPHONIC ACID COMPLEXES FOR THE TREATMENT OF CALCIFIC TUMORS

Aminophosphonic acids are known to chelate metal ions. Particularly stable chelates are formed with metals from the alkaline earth and transition metal series.

The development of a bone metastasis is a common and often catastrophic event for a cancer patient. The pain, pathological fractures, frequent neurological deficits and forced immobility caused by these metastatic lesions significantly decreases the quality of life for the cancer patient. The number of patients that contract metastatic disease is large since nearly 50% of all patients who contract breast, lung or prostate carcinoma will eventually develop bone metastases. This indicates the prevalence of the disease. Bone metastases are also seen in patients with carcinoma of the kidney, thyroid, bladder, cervix and other tumors, but collectively, these represent less than 20% of patients who develop bone metastases. Metastatic bone cancer is rarely life threatening and occasionally patients live for years following the discovery of the bone lesions. Initially, treatment goals center on relieving pain, reducing requirements for narcotic medication and increasing ambulation. Clearly, it is hoped that some of the cancers can be cured.

The use of radionuclides for treatment of cancer metastatic to the bone dates back to the early 1950's. It has been proposed to inject a radioactive particle-emitting nuclide in a suitable form for the treatment of calcific lesions. It is desirable that such nuclides be concentrated in the fast growing portion of the bone with minimal amounts reaching the soft tissue and normal bone. Radioactive phosphorus compounds (P-32 and P-33) have been proposed. However, the nuclear and biolocalization properties limit the use of these compounds. (Kaplan, E., et al, Journal of Nuclear Medicine, Vol. 1, No. 1, page 1, 1960); (U.S. Patent 3,965,254).

Another attempt has been made using phosphorus compounds containing a boron residue. The compounds were injected into the body (intravenously) and accumulated in the skeletal system. The patient was then irradiated with neutrons in order to activate the boron and give a therapeutic radiation dose. (U.S. Patent 4,399,817).

In the above-mentioned procedures, it is not possible to give therapeutic doses to the tumor without substantial damage to normal tissues. In many cases, especially for metastatic bone lesions, the tumor has spread throughout the skeletal system and amputation or irradiation of a portion of the body is not practical. (Seminars in Nuclear Medicine, Vol. IX, No. 2, April, 1979).

The use of diphosphonate complexed with Re-186 has also been proposed. (Mathieu, L. et al, Int. J. Applied Rad. & Isotopes, Vol. 30, pp. 725-727, 1979; Weinenger, J., Ketring, A. R., et al, Journal of Nuclear Medicine, Vol. 24, No. 5, page 125, 1983). However, the preparation and purification needed for this complex limits its utility and wide application.

Strontium-89 has also been proposed for patients with metastic bone lesions. However, the long half-life (50.4 days), high blood levels and low lesion to normal bone ratios limit the utility. (Firusian, N., Mellin, P., Schmidt, C. G., The Journal of Urology, Vol. 116, page 764, 1976; Schmidt, C. G., Firusian, N., Int. J. Clin, Pharmacol. 93:199-205, 1974).

A palliative treatment of bone metastases has been reported which employed I-131 labelled $\alpha$-amino-(3-iodo-4-hydroxybenzylidene)diphosphonate (Eisenhut, M., Journal of Nuclear Medicine, Vol. 25, No. 12, pp. 1356-1361, 1984). The use of radioiodine as a therapeutic radionuclide is less than desirable due to the well known tendency of iodide to localize in the thyroid. Eisenhut lists iodide as one of the possible metabolites of this compound. In addition, any I-131 left over from the iodination reaction and not separated in the washing procedure also constitutes a threat to the thyroid. Indeed Eisenhut finds 0.1% of the injected dose present in the thyroid 24 hours after injection. The high energy of the gamma ray emission from I-131 leads to inferior quality images.

Therapeutically useful complexes according to the present invention must fit certain criteria insofar as possible. It should be recognized that there are many ligands, or complexing agents, which are included by our definition of the aminophosphonic acids in which the nitrogen of the amine and the phosphorus of the phosphonic acid group are separated by an alkylene group. Many may also contain other functional groups as substituents for some, but not all, of the amine hydrogens of the ligand. It should also be recognized that the properties of the particular isotope are important. The disadvantage of any one property may be overcome by the superiority of one or more of the properties of either ligand or isotope and their combination as the complex must be considered in toto.

The following is a discussion of the criteria which must be considered in choosing any particular combination of radioisotope and ligand.

Firstly, the complex must be taken up preferentially by the bone rather than by soft tissue. Most particularly, uptake in neither liver nor bone marrow is desired. Uptake by muscle tissue, while not as damaging, is also undesirable, resulting in "inferior" imaging properties as well as unwanted dosage to non-target organs.

Another important criterion is the ratio of the amount of complex taken up by the cancerous bone to that by normal bone. High ratios are preferred since it is desired to treat the cancerous bone while irradiating the normal bone as little as possible.

The complex should be cleared from the blood rapidly for obvious reasons.

With respect to the complexed radionuclide, considerations of the nuclear properties are essential. First, the half-life must be sufficiently long to allow for localization in the bone without delivering excessive doses to non-target organs. When the complex is able to biolocalize rapidly, isotopes having shorter half-lives are useful. Isotopes with shorter half-lives provide an advantage since this permits the total dose to be given fractionally, allowing any non-target organs damaged by the radiation to recover between doses. The isotopes useful in the present invention are those which have sufficient particle emission to treat tumors.

While for successful treatment of tumors the isotope must have sufficient particle emission, it is desirable, in order to quantify the localization of the complex, that the isotope also have sufficient gamma ray production that imaging is possible. The preferred gamma ray energy should be in the 100 to 200 keV - ($16 \times 10^{-15}$ to $32 \times 10^{-15}$ joule) range although some isotopes having higher energies are potentially useful. The amount of gamma radiation should be sufficient to image, but not so great that the patient needs to be isolated to prevent him from becoming a source of radiation exposure to persons near him. It is also feasible to use isotopes which have no gamma ray production, but have sufficient particle emissions to treat the tumor successfully.

There is a need, therefore, for a system possessing the above criteria by which it is possible to deliver therapeutic radiation doses to calcific tumors with minimal doses to soft tissue or normal bone.

Such a system has now been found wherein a radioactive metal ion is complexed to a phosphorus-containing ligand. Certain of these complexes have been shown to be very selective for the skeletal system with very low soft tissue uptake. The material not taken up by bone is efficiently cleared through the kidneys into the bladder. The complexes also tend to concentrate in areas of fast-growing bone much more readily than in normal bone. The radionuclides used are particle-emitting and a high radiation dose is delivered in the area where they are deposited. Thus, therapeutic radiation doses can be delivered specifically to calcific tumors. Combinations of the various particle-emitting radionuclides can be administered for the therapeutic treatment of calcific tumors. The combinations can be complexed as herein described by complexing them simultaneously, mixing two separately complexed radionuclides, or administering two different complexed radionuclides sequentially. Combining higher beta activity radionuclides with gamma emitting radionuclides can have therapeutic advantages in not only providing the desired imaging but also in allowing for the simultaneous treatment of both large and small tumors. Also a staged treatment is possible by using at least two radionuclides having different half-lives. Particularly preferred radionuclides for the combination purposes are Sm-153 with Y-90. The main advantage to the complex of Sm-153 and Y-90 results in the ability of Sm-153 to be imaged and the higher beta activity of the Y-90. Obviously, other suitable mixtures of the complexes herein disclosed can be administered.

Particle-emitting radionuclides, of yttrium (eg. Y-90) and indium (eg. In-115) have been complexed with organic amine or substituted organic amine phosphonic acid derivatives wherein the nitrogen and phosphorus are interconnected by an alkylene or substituted alkylene group. These complexes have been found useful in the treatment of calcific tumors in animals.

The proposed use for the complexes of this invention is the therapeutic treatment of calcific tumors. These include primary tumors, where the skeletal system is the first site of involvement, and metastic bone cancer where the neoplasm spreads from other primary sites, e.g. prostate, breast, into the skeletal system. This invention provides a method of alleviating pain by delivering a therapeutic radiation dose to the aforementioned calcific tumors. This invention also provides a means of reducing the size of or destroying the calcific tumors by delivering a therapeutic radiation dose.

The organic phosphonic acid derivatives which have been found useful in complexing the particle-emitting radionuclides are organic amine or substituted organic amine compounds wherein the nitrogen and phosphorus are interconnected by an alkylene or substituted alkylene group having the formula

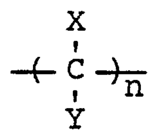

wherein X, Y are independently selected from hydrogen, hydroxyl, carboxyl, phosphonic, and hydrocarbon radicals having from 1-8 carbon atoms and physiologically acceptable salts of the acid radicals and n is 1-3 with the proviso that when n >1, each X and Y may be the same as or different from the X and Y of any other carbon atom.

The compounds can be prepared by a number of known synthetic techniques. Of particular importance is the reaction of a compound containing at least one reactive N-H group with a carbonyl compound - (aldehyde or ketone) and phosphorous acid or derivative thereof.

The following structural formulas represent some of the complexing ligands which can be used in the treatment of calcific tumors when complexed with a particle-emitting radionuclide:

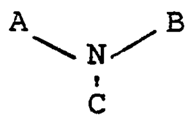

and

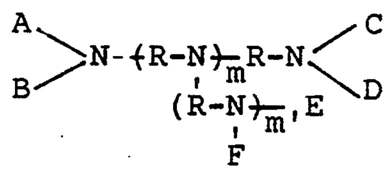

wherein substituents A, B, C, D, E and F are independently selected from hydrogen,

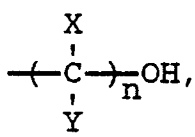
,
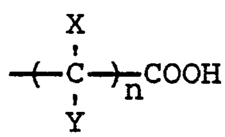
,
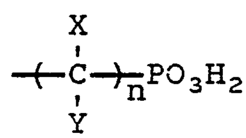
,

and physiologically acceptable salts of the acid radicals wherein X, Y, and n have been previously defined and m and m′ each is 0-10 with the proviso that at least one of said nitrogen substituents is a phosphorus-containing group and wherein R is a hydrocarbon residue which can be a linear, branched, cyclic, heterocyclic, substituted heterocyclic, or a fused ring-type structure; with the further proviso that when m or m′ >1 the E and F substituents may be the same as or different from any other substituent of any other nitrogen atom and each R can be the same as or different from any other R.

Methods for carboxyalkylating to obtain the amine derivatives containing one or more carboxyalkyl groups are well known (U.S. 3,726,912) as are the methods which give alkyl phosphonic and hydroxyalkyl - (U.S. 3,398,198) substituents on the amine nitrogens.

Some specific, but non-limiting, examples of compounds which are included by the above structures are ethylenediaminetetramethylenephosphonic acid (EDTMP), diethylenetriaminepentamethylenephosphonic acid (DTPMP), hydroxyethylethylenediaminetrimethylenephosphonic acid (HEEDTMP), nitrilotrimethylenephosphonic acid (NTMP) and tris(2-aminoethyl)aminehexamethylenephosphonic acid - (TTHMP).

Examples of particle-emitting nuclides of Yttrium and Indium useful in the practice of the invention are Y-90 and In-115.

While Y-90 and In-115 have been exemplified as the radionuclides employed with the complexing agents above, other isotopes of these elements may also be employed with the same aminophosphonic acid derivatives as disclosed herein. In addition, treatment may be carried out using a combination of the selected isotopes of Y or In, together with a complex of any other suitable radionuclide, for example one of those disclosed in EP 85302634.2, (particularly Sm-153).

The preferred embodiment of the present invention is a therapeutically effective complex of yttrium-90 - (Y-90) or indium 115 (In-115) with an aminophosphonic acid derivative selected from the group consisting of ethylenediaminetetramethylenephosphonic acid (EDTMP), diethylenetriaminepentamethylenephosphonic acid (DTPMP), hydroxyethylethylenediaminetrimethylenephosphonic acid (HEEDTMP), nitrilotrimethylenephosphonic acid (NTMP) and tris(2-aminoethyl)aminehexamethylenephosphonic acid - (TTHMP).

For the purpose of convenience the abbreviations given in the parentheses above will be used to denote the respective radionuclides and aminophosphonic acid derivatives hereinafter.

A more preferred embodiment of the present invention is a therapeutically effective complex of a particle-emitting radionuclide selected from the group consisting of Gd-159, Lu-177, Sm-153, and Yb-175 with an aminophosphonic acid derivative selected from the group consisting of EDTMP, DTPMP, HEEDTMP, NTMP and TTHMP.

Particularly preferred embodiment of the present invention is a therapeutically effective complex of yttrium-90 (Y-90) or indium 115 (In-115) with EDTMP, DTPMP, HEEDTMP, NTMP or TTHMP.

Particularly, the invention includes within its scope a complex of Y-90 or In-115, together with a complex of Sm-153 with an aminophosphonic acid derivative which is EDTMP, DTPMP, HEEDTMP, or TTHMP.

For the purpose of the present invention, therapeutically effective complexes described herein and physiologically-acceptable salts thereof are considered equivalent. Physiologically-acceptable salts refer to the acid addition salts of those bases which will form a salt with at least one acid group of the complex and which will not cause an adverse physiological effect when administered to an animal at dosages consistent with good pharmacological activity. Suitable bases include, for example, the alkali metal and alkaline earth metal hydroxides, carbonates, and bicarbonates such as sodium hydroxide, potassium hydroxide, calcium hydroxide, potassium carbonate, sodium bicarbonate, magnesium carbonate and the like, ammonia, primary, secondary and tertiary amines and the like. Physiologically-acceptable salts of the present invention may be prepared by treating the complex having at least one acid group with an appropriate base.

Radionuclides can be produced in several ways. In a reactor, a nuclide is bombarded with neutrons to obtain a nuclide with additional neutrons in its nucleus.

e.g. Sm-152 + neutron → Sm-153 + gamma

Another method of obtaining radioisotopes is by bombarding nuclides with linear accelerator or cyclotron-produced particles. Yet another way of obtaining radioisotopes is to isolate them from fission product mixtures. The method of obtaining the radionuclide is not critical to the present invention.

When aqueous solutions of metal ions are mixed with solutions containing complexing agents, such as those described in this invention, a complex between the metal ion and the ligand can be formed as shown by the equation below.

$$M + L \rightleftarrows M \bullet L$$

The reaction is believed to be an equilibrium such that the concentrations of metal (M) and complexing agent (L) can affect the concentration of species present in solution. Competing side reactions, such as metal hydroxide formation, can also occur in aqueous solution.

$$xM + yOH^- \rightarrow M_x(OH)_y$$

The $OH^-$ concentration in solution which is related to pH is, therefore, an important parameter to be considered. If the pH is too high, the metal tends to form metal hydroxides rather than complexes. The complexing agents may also be affected by low pH. Complexation may require the loss of protons; therefore at low pH, conditions may not be favorable for complexation to occur. The complexes of this invention can be formed at several pH's. Consideration must be given to the solubility characteristics of the ligand, radionuclide, and complex. Although complexation will occur at other pH values, pH from 5-11 is preferred for complexation.

The metal and ligand may be combined under any conditions which allow the two to form a complex. Generally, mixing in water at a controlled pH (the choice of pH is dependent upon the choice of ligand and metal) is all that is required. In some cases heating may be required to obtain maximum complex yield.

The ratio of ligand to metal is a result of two competing considerations. As indicated above the ligand and metal are believed to be in equilibrium with the complex. On the one hand, it is desirable to have a large quantity of ligand (L), so that there is a minimum amount of free metal (M), because the uncomplexed metal may cause serious side effects in the patient. On the other hand, excess free ligand may compete for sites in the target, thus rendering the treatment less effective. The fact that the ligands within the scope of the invention are capable of complexing with varying molar ratios of metal adds a degree of complexity to these considerations. Although it is difficult to generalize, it is believed that the ligand to metal molar ratio is desirably 0.1:1 to 3000:1, preferably 1:1 to 2000:1, more preferably 1:1 to 1000:1.

The samarium used in the following example was either natural $Sm_2O_3$ (99.9 percent from Spex Industries) or isotopically enriched (99.06 percent Sm-152) $Sm_2O_3$.

The Sm-153 used in this study was produced by neutron irradiation at the University of Missouri Research Reactor. Preliminary studies were carried out using Sm-153 produced by short (5-30 minutes) irradiations of natural $Sm_2O_3$, in the reactor's pneumatic tube system. The specific activity of Sm-153 produced by this method was 0.5-3.0 Ci/g (18.5 to 111 Gbq/g).

The majority of this work was carried out using Sm-153 produced by irradiating 99.06 percent enriched $^{152}Sm_2O_3$ in the first row reflector at a neutron flux of 1 x $10^{14}$ neutron/cm$^2$•sec. Irradiations were generally carried out for 50-60 hours, yielding a Sm-153 specific activity of 1000 -1300 Ci/g (37 x $10^3$ to 48.1 x $10^3$ GBq/g).

To irradiate $Sm_2O_3$ for production of Sm-153, the desired amount of target was first weighed into a quartz vial, the vial flame sealed under vacuum and welded into an aluminum can. The can was irradiated for the desired length of time, cooled for several hours and opened remotely in a hot cell. The quartz vial was removed and transferred to a glove box, crushed into a glass vial which was then sealed with a rubber septum and an aluminum crimp cap. One milliliter of 1-4 M HCl was then added to the vial via syringe to dissolve the $Sm_2O_3$. Once dissolved, the solution was diluted to the appropriate volume by addition of water. The solution was removed from the original dissolution vial which contains the chards of the crushed quartz vial, and transferred via syringe to a clean glass serum vial. This solution was then used for complex preparation. Similar procedures were used to prepare other radionuclides, e.g. Y-90 and In-115.

The various complexes employed in this invention were prepared as follows: the desired amount of ligand was placed in a vial and dissolved by addition of water. At some higher ligand concentrations, it was necessary to add base in order to completely dissolve the ligand. Heating was also found to be useful for dissolving the ligands. The appropriate amount of the samarium or other radionuclides in the stock solution described above was then added to the ligand solution. The pH of the resulting solution was then raised to the appropriate level by addition of NaOH. The solution was heated to 60°-70°C for 30 minutes in a water bath to insure maximum complex formation. The pH of the solution was then adjusted to 7-8 by addition of 1 M HCl.

The complex yield was determined by placing 5-20 microliters (depending on activity) of the complex solution onto a 0.5 cm$^3$ column of a commercially available synthetic organic cation exchange resin. The column was then eluted with two separate 10 milliliter volumes of isotonic saline. The anionic complexes are not retained by the resin and were eluted by the saline solution, while any uncomplexed metal was retained on the column. The eluant solutions and the column were then counted for the characteristic emission of the particular radionuclide, e.g. 103 keV (16.5 x $10^{-15}$ joule) gamma ray of Sm-153. The complex yield was obtained by adding the counts in the eluant solutions and dividing by the total of the eluant solutions and the column.

This invention provides a means of delivering a therapeutic radiation dose to calcific tumors. It may also be desirable in the cases where the radionuclide has imageable gamma photons to inject a "sub-therapeutic" dose and determine the fate of the radionuclide using a scintillation camera prior to injecting a therapeutic radiation dose. Therefore, the levels of radiation injected could be as low as 1 mCi (37 MBq) for imaging prior to the therapy. Therapeutic doses will be greater. The does to the tumor may range from 100 to 10,000 rads (1 Gy to 100 Gy). The preferred dose to the tumor ranges from 1,000 to 8,000 rads (10 Gy to 80 Gy). For complexes such as Sm-153-EDTMP amounts ranging from 0.1 mCi/kg body weight to 3 mCi/kg body weight are preferred. The amount of activity required to deliver a therapeutic dose may vary with the individual radionuclides. Individual doses may be given in one injection or fractionated into several injections totaling the aforementioned dose per treatment.

Biodistribution of various complexes of the present invention was studied in rats and rabbits.

Studies to determine the qualitative distribution of the various complexes of the present invention were conducted by injecting the complexes into rats and obtaining the gamma ray images of the entire animal at various times up to two hours after injection.

Male Spague Dawley rats were anesthetized by injection with sodium pentabarbitol and the jugular vein cannulated. The animals were then injected with 50 to 150 microliters of the various complexes and gamma ray images were taken on a Nuclear Chicago® Pho-Gamma® II scintillation camera immediately after injection and approximately every half hour thereafter for two hours.

Quantitative biodistributions were obtained by injecting 50-100 microliters of the complex solution into the tail vein of unanesthetized male Spague Dawley rats. The rats were then placed in cages lined with absorbent paper in order to collect all urine excreted prior to sacrifice. After two hours, the rats were sacrificed by cervical dislocation and the various tissues dissected out. The samples were then rinsed with saline, blotted dry on absorbent paper and weighed. The samples were counted with a NaI uptake counter approximately one foot from the face of the crystal, in order to minimize geometry differences between the different size samples.

To minimize differences in age between groups of animals, all rats used were in the 160-220 gram weight range. All animals were kept "in-house" for at least one week prior to use to minimize the effects of stress that occur during shipping.

The various complexes of the present invention were also evaluated in rabbits. The animals used in these studies were male New Zealand white rabbits in the 2.6 to 3.2 kilogram weight range. The animals were kept "in-house" for at least a week prior to use.

The rabbits were injected with 100-250 microliters of the complex solution via a cannula placed in the marginal ear vein. In studies where blood clearance was measured, blood samples were taken through a heparinized cannula placed in the marginal vein of the ear not used for injection of the complex.

Three hours after injection, a blood sample was taken by cardiac puncture and the animal was then sacrificed by injection of a commercial euthanasia solution. After sacrifice, images were obtained by placing the carcass directly on the face of a large field of view scintillation camera.

Uptake of the complexes in the lesion compared to uptake in normal bone, or lesion/normal bone ratios, is a particularly important parameter for calculating the complex suitability as a therapeutic agent. To determine the lesion to normal bone ratios, a modified drill hole method (Subramanian, G. et al., 19th lit. Annual Meetings of S.N.M., Bern, Switzerland, September 8-11, 1981) was used.

In order to simulate uptake in rapidly growing bone, such as that found in cancerous bone, two holes were drilled into the surface of the tibia of a rabbit in order to damage the bone. Seven to ten days later, the animal was injected with the complex. After three hours, the animal was anesthetized and imaged using an Anger camera and pinhole collimator.

The following examples are illustrative of the present invention and are not to be construed as limiting the scope thereof.

Example 1

Into a suitable reaction vessel equipped with a thermometer, magnetic stirring bar, dropping funnel, and an atmosphere of nitrogen were charged phosphorous acid (94.5 g) and degassed water (100 ml). Dissolution of the phosphorous acid was achieved by stirring and the solution was then treated with concentrated hydrochloric acid (112 ml). The dropping funnel was charged with ethylenediamine (15 g) and adjusted to allow dropwise addition of the diamine to the acidic solution. When addition was complete a heating mantle was installed and the solution refluxed for one hour. At the end of this time the dropping funnel was charged with formaldehyde (85 g of a 37% aqueous solution) which was added dropwise over a two-hour period with continued heating to maintain reflux during the addition. After all the formaldehyde was added, the reaction mixture was stirred under reflux for an additional two hours, then allowed to cool slowly overnight during which time the product precipitated. Vacuum filtration followed by cold water washing yielded ethylenediaminetetramethylenephosphonic acid (EDTMP).

Example 2

35 to 45 milligrams of EDTMP prepared in Example 1 was weighed into a vial and dissolved using 0.75 ml of distilled water. To this, 0.25 -0.75 ml of Y-90 in dilute HCl was added. The pH of the resulting solution was then adjusted to 10 by addition of NaOH. The resulting solution was heated to 60°C -70°C for 30 minutes in a water bath to ensure maximum complex formation. The pH of the solution was then adjusted to 7-8 by addition of 1 M HCl. The yield of the complex was over 95 percent.

Example 3

Into a suitable reaction vessel equipped with a thermometer, magnetic stirring bar, dropping funnel, and an atmosphere of nitrogen were charged phosphorous acid (94.5g) and degassed water (100 ml). Dissolution of the phosphorous acid was achieved by stirring and the solution was then treated with concentrated hydrochloric acid (112 ml). The dropping funnel was charged with diethylenetriamine (20.6 g) and adjusted to allow dropwise addition of the amine to the acidic solution. When addition was complete a heating mantle was installed and the solution refluxed for one hour. At the end of this time the dropping funnel was charged with formaldehyde (85 g of a 37% aqueous solution) which was added dropwise over a two-hour period with continued heating to maintain reflux during the addition. After all the formaldehyde was added, the reaction mixture was stirred under reflux for an additional two hours, then allowed to cool. Diethylenetriaminepentamethylenephosphonic acid (DTPMP) was isolated from the reaction mixture.

Example 4

70 mg of EDTMP prepared in Example 1 was weighed into a vial and dissolved using 1 ml of normal saline with a few drops of concentrated $NH_4OH$. To this solution, 1 ml of freshly eluted In-115 solution was added and the pH was adjusted to 9 using dilute NaOH and HCL. 1 to 5 $\mu$l of the resultant solution was placed on a column containing a cation exchange resin and eluted with 10 ml of saline solution. The amount of free In in solution was calculated as the amount left on the column divided by the total activity X 100%. By this method, the yield of the complex was over 97%.

Example 5

Into a suitable reaction vessel equipped with a thermometer, magnetic stirring bar, dropping funnel, and an atmosphere of nitrogen were charged phosphorous acid (94.5 g) and degassed water (100 ml). Dissolution of the phosphorous acid was achieved by stirring and the solution was then treated with concentrated hydrochloric acid (112 ml). The dropping funnel was charged with N-hydroxyethylethylenediamine (34.6 g) and adjusted to allow dropwise addition of the diamine to the acidic solution. When addition was complete a heating mantle was installed and the solution refluxed for one hour. At the end of this time the dropping funnel was charged with formaldehyde (85 g of a 37% aqueous solution) which was added dropwise over a two hour period with continued heating to maintain reflux during the addition. After all the formaldehyde was added, the reaction mixture was stirred under reflux for an additional two hours, then allowed to cool. Hydroxyethylethylenediaminetrimethylenephosphonic acid - (HEEDTMP) was isolated from the reaction mixture.

Example 6

70 mg of HEEDTMP prepared according to Example 5 was weighed into a vial and dissolved using 1 ml of normal saline solution by adding several drops of concentrated $NH_4OH$. To this solution, 1 ml of freshly eluted In-115 solution was added and the pH was adjusted to 9 using dilute NaOH and HCl. 1 to 5 $\mu$l of the resultant solution was placed on a column containing a cation exchange resin and eluted with 10 ml of saline solution. The amount of free In in solution was calculated as the amount left on the column divided by the total activity X 100%. By this method, the yield of the complex was over 97%.

Example 7

70 mg of DTPMP prepared according to Example 3 was weighed into a vial and dissolved using 1 ml of normal saline solution by adding several drops of concentrated $NH_4OH$. To this solution, 1 ml of freshly eluted In-115 solution was added and the pH was adjusted to 9 using dilute NaOH and HCl. 1 to 5 $\mu$l of the resultant solution was placed on a column containing a cation exchange resin and eluted with 10 ml of saline solution. The amount of free In in solution was calculated as the amount left on the column divided by the total activity X 100%. By this method, the yield of the complex was over 97%.

Laboratory rats were injected with the above complex from Examples 2 and 4 (50 - 75 ul) via the tail vein. After two hours, the animals were sacrificed by cervical dislocation and organs and tissues removed. Samples were counted with a NaI uptake counter to determine the biolocalization of the complex. It was found that a significant amount of the activity was concentrated in the skeletal system with very little soft tissue uptake.

Y-90 does not emit gamma photons. To avoid the difficulties involved in counting betas, the Bremsstrahlung radiation was measured. This radiation is caused by the acceleration of a negatively charged eletron by a positive nucleus. The result is a continuum of electromagnetic radiation ranging in energy from 0 to the energy of the beta emitted, with higher abundance of photons in the low energy end of the spectrum. The bio-distribution data is shown in Table I.

TABLE I

| % Dose in: | Example Nos. 2 | 4 | 6 | 7 |
|---|---|---|---|---|
| Skeleton* | 56±9 | 53±6 | 44±3 | 46±7 |
| Liver | 0.07±0.07 | 0.2±0.7 | 0.3±0.1 | 0.1±0.05 |
| Muscle | 0.2±0.2 | 1.2±0.1 | 1.6±0.3 | 1.0±0.6 |
| Blood | N.A. | 0.7±0.3 | 1.3±0.1 | 0.3±0.2 |
| Bone/Blood | 45 | 54 | 27 | 43 |
| Bone/Muscle | 120 | 217 | 138 | 210 |

*Based on % Dose in Femur X 25

Number of Rats Tested = 5

N.A. = Not Available

Comparative Example

Two commercially available non-nitrogen-containing phosphonic acid compounds which are well known as diagnostically useful compounds when complexed with Tc-99m were complexed with Sm-153 and tested in the manner of the complexes of the present invention. The data comparable to that given for the examples of the invention is shown in Table A below:

TABLE A. Two Hour Biodistribution of Complexes in Rats

| | $^{153}$Sm-MDP*** | $^{153}$Sm-HEDP* |
|---|---|---|
| % Dose in Skeleton** | 1.65 ±0.43 | 20.5 ±1.4 |
| Blood | 0.23 ±0.19 | 13.1 ±2.6 |
| Liver | 84.5 ±3.8 | 3.5 ±0.9 |
| Kidney | 0.45 ±0.19 | 0.99 ±0.44 |
| Urine | 0.20 ±0.14 | 41.3 ±4.6 |
| Bone/Blood | 8.6 ±5.0 | 1.3 ±0.3 |
| Bone/Muscle | 7.1 ±3.2 | 10:.5 ±2.9 |

Number of rats tested = 5

* 1-hydroxyethylidine-1,1-diphosphonic acid (HEDP)

** Based on % dose in femur X 25

***Methylenediphosphonate

It should be noted that the comparative examples in above Table A are of two non-nitrogen-containing phosphonic acid complexes. These have certain undesirable properties which make them inferior to the complexes of the invention. Thus, the Sm-MDP shows high liver uptake while the Sm-HEDP has both low skeletal uptake and poor clearance from the blood.

## Claims

1. A therapeutically effective complex of a paticle-emitting radionuclide of yttrium or indium with an aminophosphonic acid derivative which is ethylene-diaminetetramethylenephosphonic acid (EDTMP), diethylenetriaminepentamethylenephosphonic acid (DTPMP), hydroxyethylethylenediaminetrimethylenephosphonic acid (HEEDTMP), nitrilotrimethylenephosphonic acid - (NTMP) or tris(2-aminoethyl) aminehexamethylenephosphonic acid (TTHMP) or a physiologically acceptable salt of such a complex.

2. A complex as claimed in Claim 1, characterized in that said radionuclide is Y-90.

3. A complex as claimed in Claim 1, characterized in that said radionuclide is In-115.

4. A complex as claimed any one of the preceding claims, which also comprises an additional rare earth radionuclide.

5. A complex as claimed in Claim 4, wherein the additional rare earth radionuclide is an isotope of gadolinium, holmium, lutetium, samarium or ytterbium.

6. A complex as claimed in Claim 5, wherein the additional rare earth radionuclide is gadolinium-159 - (Gd-159), holmium-166 (Ho-166), lutetium-177 (Lu-177), samarium-153 (Sm-153) or ytterbium-175 (Yb-175).

7. A complex as claimed in Claim 6, wherein the additional radionuclide is Sm-153.

8. A complex as claimed in any one of Claims 1 to 7, characterized in that the aminophosphonic acid derivative is ethylenediaminetetramethylenephosphonic acid (EDTMP).

9. A complex as claimed in any one of Claims 1 to 7, characterized in that the aminophosphonic acid derivative is diethylenetriaminepentamethylenephosphonic acid (DTPMP).

10. A complex as claimed in any one of Claims 1 to 7, characterized in that the aminophosphonic acid derivative is hydroxyethylethylenediaminetrimethylene phosphonic acid (HEEDTMP).

11. A complex as claimed in any one of Claims 1 to 7, characterized in that said aminophosphonic acid derivative is nitrilotrimethylenephosphonic acid (NTMP).

12. A complex as claimed in any one of Claims 1 to 7, characterized in that the aminophosphonic acid derivative is tris(2-aminoethyl) aminehexamethylenephosphonic acid (TTHMP)>

13. A process for the preparation of a therapeutically effective complex as claimed in Claim 1, characterized in that a radionuclide which is Y-90 or In-115 is contacted with an aminophosphonic acid derivative which is EDTMP, DTPMP, HEEDTMP, NTMP or TTHMP.

14. A process as claimed in Claim 13, wherein the said contact is carried out in the presence of water at a pH of from 5 to 11.

15. A process as claimed in Claim 13 or Claim 14, wherein the said contact is carried out at elevated temperature.

16. A process as claimed in any one of Claims 13 to 15, including the step of adjusting the pH of the complex to from 7 to 8.

17. A process as claimed in any one of Claims 13 to 16, characterized in that said aminophosphonic acid derivative is EDTMP.

18. A process as claimed in any one of Claims 13 to 16, characterized in that the aminophosphonic acid derivative is DTPMP.

19. A process as claimed in any one of Claims 13 to 16, characterized in that the aminophosphonic acid derivative is HEEDTMP.

20. A process as claimed in any one of Claims 13 to 16, characterized in that the aminophosphonic acid derivative is NTMP.

21. A process as claimed in any one of Claims 13 to 16, characterized in that the aminophosphonic acid derivative is TTHMP.

22. A process as claimed in any one of Claims 13 to 16, characterized in that the aminophosphonic and said radionuclide acid derivative are contacted in a molar ratio of from 0.1:1 to 3000:1.

23. A complex as claimed in any one of Claims 1 to 12, for use in the treatment of cancer.

24. The use of a complex as claimed in any one of Claims 1 to 12, in the manufacture of a medicament for the treatment of cancer.

European Patent Office

# EUROPEAN SEARCH REPORT

Application number

EP 85 30 8772

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| E | EP-A-0 164 843 (THE DOW CHEMICAL COMP.) * Page 3, line 26 - page 17, line 19 * | 4-12, 24 | A 61 K 43/00<br>C 07 F 9/38<br>C 07 B 59/00 |
| X | CHEMICAL ABSTRACTS, vol. 93, no. 17, 27th October 1980, page 285, abstract no. 163556v, Columbus, Ohio, US; JI-XIAO MA et al.: "Indium-113m-labeled bone imaging agents - animal experiment and clinical application of 113m In-DTPMP and 113m In-EDTMP", & CHUNG-HUA I HSUEH TSA CHIH (PEKING) 1980, 60(4), 200-3 * Abstract * | 1,3,8, 9,24 | |
| X | CHEMICAL ABSTRACTS, vol. 95, no. 23, 7th December 1981, page 305, abstract no. 199803d, Columbus, Ohio, US; C.M. HSIA et al.: "Preparation of 113m In-DTPMP bone scanning agent and its preliminary clinical application", & CHUNG-HUA HEH I HSUEH TSA CHIH 1981, 1(1), 7-11 * Abstract * | 1,3,9, 13-15, 18,24 | TECHNICAL FIELDS SEARCHED (Int. Cl.4)<br>A 61 K |

-----

The present search report has been drawn up for all claims

| Place of search | Date of completion of the search | Examiner |
|---|---|---|
| THE HAGUE | 15-07-1986 | REMPP G.L.E. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82